# EUROPEAN PATENT SPECIFICATION

(11) **EP 2 869 795 B1**
(45) Date of publication and mention of the grant of the patent: **06.04.2016**
(21) Application number: 13727993.1
(22) Date of filing: 24.04.2013
(51) Int. Cl.: A61F 5/02, A41D 13/05

(54) **GIRDLE**
KORSETT
GAINE

(30) Priority: 03.07.2012 IT MI20121169
(43) Date of publication of application: 13.05.2015
(73) Proprietor: Indaco S.r.l., 85020 Filiano PZ (IT)
(72) Inventor: FRANGI, Massimo, I-57028 Suvereto LI (IT); FRANGI, Roberta, I-21020 Buguggiate VA (IT)
(74) Representative: Perani, Aurelio
(86) International application number: PCT/IB2013/053227
(87) International publication number: WO 2014/006521

(56) References cited:
- WO-A1-2006/119827

## Description

### TECHNICAL FIELD

The present invention relates to a girdle for use in orthopedics for injury rehabilitation and prevention or in other fields (i.e. in sports) for prevention of lower spine and lumbar and abdominal injuries of a user.

### BACKGROUND OF THE INVENTION

Girdles have been known for years and are composed of a belt, usually elastic, which is designed to encircle the waist of a user to assist the spine and the lumbar and abdominal muscles in torso support.

Especially in therapeutic applications, girdles are required to encircle and adequately support the waist of the user. For this reason, a plurality of sizes (which may be as many as ten) shall be provided for each type of girdle, for the user to select the one that best fits his/her build. Of course, if the user's build changes (i.e. due to weight loss or increase) during use of the girdle, a girdle of a different size might have be purchased. Furthermore, girdle manufacturers shall be able to ensure availability of all sizes, which increases manufacturing and logistics costs.

Another important characteristic of girdles is the possibility of making adjustments such that the girdle perfectly fits the anatomy of the user. Indeed, even people of the same size have their own particular anatomy (e.g. more or less prominent hips, lower or higher waist lines, and so on). The possibility of making adjustments should be further combined with the possibility of easily wearing the girdle.. In this respect, WO2006119827 discloses a corset comprising two substantially identical portions, each having a plurality of laces extending therefrom at the back portion (i.e. the one designed to be located near the spine of the user) of the corset. The laces that extend from the first portion of the corset cross with those that extend from the second portion (and vice versa) and are gathered into two groups, each connected to a loop engaged by a respective belt for tensioning the second portion of the corset. Each pair of belts for tensioning a portion of the corset (an upper portion and a lower portion) can be also released from the other portion of the corset, when the latter encircles the waist of user. Thus, the two corset portions are joined together, and as the tensioning belts are tightened or loosened when the corset is being closed, corset compression may be increased or released. Also, by setting different tensions for the upper and lower tensioning belts, different compression degrees and lengths may be obtained for the lower and upper portions of the corset, whereby the latter can be conformed to different anatomies.

While this prior art corset solves most of the above mentioned problems, it still suffers from certain drawbacks.

In order to obtain different compression degrees and lengths for the upper and lower portions of the corset while maintaining an adequate tensile and structural symmetry in the corset, the tension of tensioning belts should be accurately calibrated when the corset is being closed, which would require repeated tension adjustments for the upper and lower belts. This cannot be always done easily, whereby the corset does not always perfectly fit the anatomy of the user.

Furthermore, the force that the user is required to apply on the tensioning belts to close the corset is rather important, though not excessive. Therefore, older or physically impaired people might not be independent in wearing the corset. In the light of the above prior art, the technical purpose of the present invention is to provide a girdle that obviates the above mentioned drawbacks.

Particularly, the object of the present invention is to provide a girdle that affords easy differentiated adjustment of the upper and lower portions of the girdle.

A further object of the present invention is to provide a girdle that can be easily used by elder and physically impaired users.

Another object of the present invention is to provide a girdle that can cover a wide range of sizes.

Yet another object of the present invention is to provide a girdle that can fit the, peculiar anatomy of the user.

### SUMMARY OF THE INVENTION

According to the present invention, the technical purpose and the intended objects are fulfilled by a corset having the features of one or more of the annexed claims.

### BRIEF DESCRIPTION OF THE DRAWINGS

The features and advantages of the invention will appear from the following detailed description of one embodiment, which is illustrated without limitation in the annexed drawings, in which:
- Figure 1 shows a plan view of a girdle of the present invention, in a first operating configuration,
- Figure 2 shows the girdle of Figure 1 in a second operating configuration;
- Figure 3 is a perspective view of the girdle of Figure 1, with certain parts omitted to better show other parts; and
- Figures 4, 5 and 6 are schematic views of the steps for wearing the girdle of Figure 1.

### DETAILED DESCRIPTION OF THE INVENTION:

A girdle according to the present invention has been generally designated by numeral 1.

The girdle 1 comprises a first portion 2 and a second portion 3, each in band form. The two portions 2, 3 are made of a flexible, non-elastic material, for them to conform to the curves of a user, without elastically extending to an appreciable or considerable extent. The two portions 2, 3 have free ends 2a, 3a, that are able to be removably fastened together. As used herein, the term removably fastened is intended to indicate a connection between two elements that may be made and effected by a user and may be removed by the user him/herself. In the preferred embodiment of the invention, such type of fastening arrangement is given by a hook fabric area disposed on one of the two elements to be fastened, and designed for connection to a corresponding area of the other element that is made of a hairy fabric (or vice versa), to form a Velcro® connection.

A first plurality of strips 4 and a second plurality of strips 5 extend from respective ends 2b, 3b of the first and second portions 2, 3 opposite to the above mentioned free ends 2a, 3b. The first plurality of strips 4 extends toward the second portion 3 and the second plurality of strips 5 extends toward the first portion 2. These strips are also made from a flexible, non-elastic material. The ends of the strips of the first 4 and second 5 pluralities gather into at least first 6 and second 7 groups respectively, each being joined to respective first 8 and second 9 joining members.

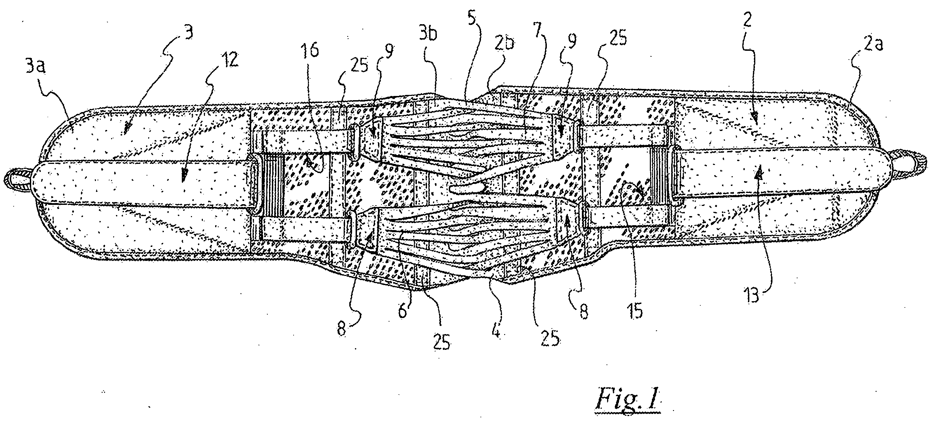

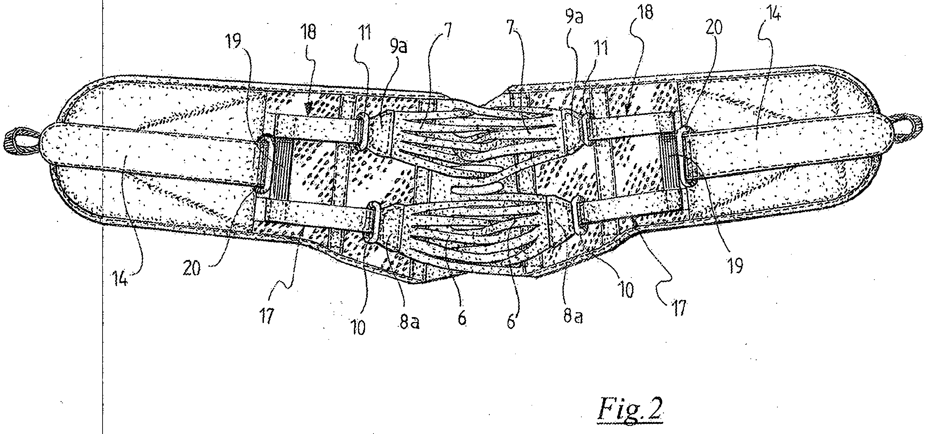

In the preferred embodiment of the invention, two first joining members 8 and two second 9 joining members are provided. The first joining members 8 are placed at the lower portion of the girdle (the one that is proximal to the pelvis of the user) and the second joining members 9 are placed at the upper portion of the girdle (the one that is distal from the pelvis of the user),as shown in Figure 1. Therefore, a first 8 and a second 9 joining members are both subtended by the first portion 2 of the girdle, as well as by the second portion 3 of the girdle. It shall be noted that the strips of the first plurality of strips 4 alternate (by crossing) with the strips of the second plurality of strips 5, such that the strips of the first plurality cannot disengage from those of the second plurality and vice versa, thereby mutually fastening the two portions 2, 3 of the girdle. The joining members 8, 9 include a retaining portion 8a, 9a (see Figure 2) in which the ends of the respective groups of strips are firmly fastened together. The joining members 8, 9 further include respective loops 10, 11, which are designed to slidingly engage a respective strap 17, 18, a better described hereinafter. These loops 10, 11 are firmly fastened to the retaining portions 8a, 9a and extend away therefrom.

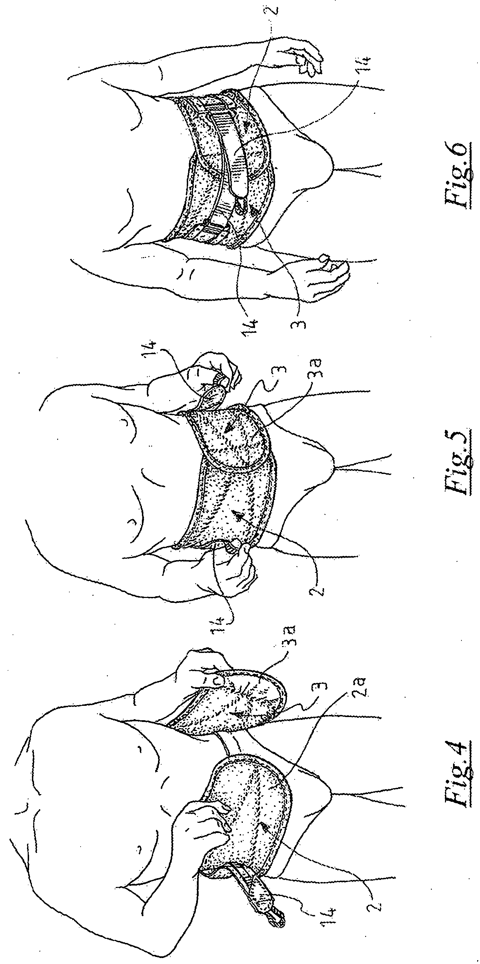

The girdle 1 further comprises tensioning members 12, 13, each being fastened to one of the two portions 2, 3 of the girdle. Each tensioning member 12, 13 is also adapted to be removably fastened (as described above) to the portion 2, 3 of the girdle to which it is not permanently fastened, and operates on the first 8 and second 9 joining members that are subtended by the portion 2, 3 to which it is permanently fastened. Namely, a first tensioning member 12, 13 is permanently fastened to the first portion 2 of the girdle, and adapted to be removably fastened to the second portion 3, and operates on the first 8 and second 9 joining members that are subtended by the first portion 2 of the girdle. Likewise, a second tensioning member 13, 13 is permanently fastened to the second portion 3 of the girdle, and adapted to be removably fastened to the first portion 3, and operates on the first 8 and second 9 joining members that are subtended by the second portion 3 of the girdle. The tensioning members are also made from a flexible, non-elastic material. In the preferred embodiment of the invention, there are two tensioning members. Particularly, each tensioning member comprises a strap 14 (see Figures 2 and 4) having a first end 14a fastened to its respective girdle portion and a second end 14b designed to be held by a user. The second end 14b is adapted to be removably fastened to a corresponding area of a girdle portion or the other strap. By actuating the straps 14, the user may encircle the girdle around his/her waist, as schematically shown in Figures 5 and 6.

Advantageously, the girdle comprises pretensioning members 15, 16 for connecting the first and second joining members 8, 9 to their respective tensioning members 12, 13. At least the pretensioning members 15, 16 that connect the first 8 or the second joining members 9 to their tensioning members 12, 13 can be removably fastened to the first 2 and second 3 portions respectively in band form. The pretensioning members 15, 16 that are not designed to be removably fastened to the first 2 an second 3 portions respectively of the girdle are stably and irremovably fastened to the first 2 and second 3 portions respectively of the girdle. Thus, as more clearly shown hereinafter, the girdle can fit the particular anatomy (hip width relative to the waist) of the user. In the preferred embodiment of the invention, as shown in the figures, all the pretensioning members 15, 16 are adapted to be removably fastened to the first 2 and second 3 portions of the girdle respectively. Thus, the user may fasten the pretensioning members 15, 16 in different positions on the portions 2, 3 of the girdle, thereby providing different tensioning degrees on the plurality of strips 4, 5, reflecting into different relative positions of the first 2 and second 3 positions of the girdle, such that the girdle may be configured before being worn. Indeed, as mentioned above, the pretensioning members 15, 16 interact with the joining members 8, 9 which are stably fastened.to the groups 6, 7 of pluralities of strips 4, 5, thereby interacting with the pluralities of strips 4, 5. Furthermore, as mentioned above, as the girdle is being closed the joining members 8, 9 are actuated by a respective tensioning member 12, 13. Since the pretensioning members 15, 16 also interact with the latter, the above described preconfiguration of the girdle is not altered as the girdle is being worn. In other words, the pretensioning members 15, 16 are configured as transfer members between the tensioning members 12, 13 and the joining members 8, 9, thereby allowing preconfiguration of the relative positions of the two portions 2, 3 of the girdle by adjusting the relative distance between each joining member 8, 9 and its respective tensioning member 12, 14 (as more clearly shown hereinafter). The pretensioning members are also made from a flexible, non-elastic material.

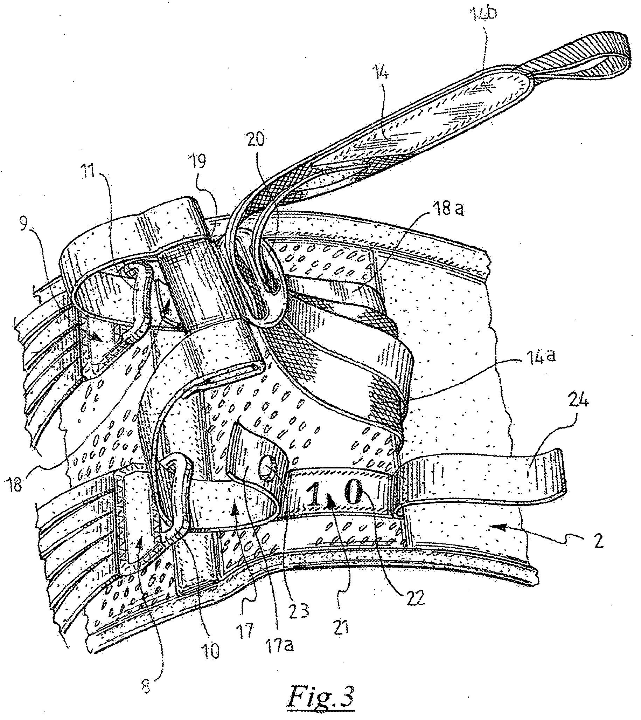

In the preferred embodiment of the invention, each pretensioning member comprises first 17 and second 18 straps, each having a free end 17a, 18a (see Figures 2 and 3). The free ends 17a, 18a of the straps 17, 18 are adapted to be removably fastened to the first or second portion of the girdle respectively, as shown in Figure 3, which shows the free end 17a of the first strap 17 as it is fastened. Opposite to its free end, each strap 17, 18 is fastened to a joining element 19 that integrally joins the two straps 17, 18. A loop 20 is applied to the joining element 19, and slidingly receives the strap 14 that defines a respective tensioning member. In an intermediate position between the free end 17a, 18a and the joining element 19, each strap is slidingly inserted in the loop 10, 11 of the joining elements 8, 9.

It shall be noted that the two straps 17, 18 and the joining element 19 allow the strap 14 to act upon two groups of pluralities of strips 4, 5. Thus, the user may act on one element only, i.e. a strap 14, to stretch both groups of strips 6, 7, which will considerably facilitate the girdle wearing process. Furthermore, as mentioned above, the straps 17, 18 allow preconfiguration of the mutual orientation of the two portions 2, 3 of the girdle. This will also allow both the two portions 2, 3 to be moved toward or away from each other (for preconfiguration of the girdle in various sizes), and the upper portion of the girdle to be tightened or loosened relative to the lower portion (for fitting the girdle to the particular anatomy of the user). Furthermore, these steps are advantageously carried out before wearing the girdle, and hence may be performed in a very easy and convenient manner.
For easier fastening of the straps 17, 18, indexing members 21 are provided (see Figure 3). These indexing members 21 include a graded scale 22 (given by progressive numbers in Figure 3), representative of the degree of pretensioning of the pluralities of strips. For accurate positioning of the strap on the selected index, each strap has a hole 23 (see Figure 3), which allows viewing of the index of the graded scale 22 on which the strap has been fastened. Furthermore, in order to prevent the strap from being released from the selected position due to accidental shocks, an appendage 24 is designed to be removably fastened to the upper surface (i.e. the surface opposite to the one that engages the graded scale) of the strap. This appendage 24 is firmly fastened at one end to the corresponding portion 2, 3 of the girdle. When the straps are placed at the same indices of the grades scale, the first groups of strips 6 and the second groups of strips 8 are pretensioned to the same extent. This will allow the girdle to fit to size, i.e. to be adjusted according to the user's size. When the straps 17, 18 engage different indices of the graded scale, the upper portion of the girdle is loosened or tightened relative to the lower portion, whereby the girdle is adjusted according to the anatomy of the user (more or less narrow hips, more or less pronounced belly).

For perfect support of the user body in the region in contact with the girdle, the latter comprises a plurality of stiffening shafts 25 in pockets formed on the first 2 and second 3 portions of the girdle. Advantageously, all the stiffening shafts 25 are located between their respective girdle portion and the pretensioning members 15, 16 and/or the pluralities of strips 4, 5. Thus, when the girdle is worn, the stiffening shafts 25 are pressed in position by the pretensioning members 15, 16 themselves and/or by the pluralities of strips 4, 5.

In operation, the pretensioning steps as described above and schematically shown in Figure 3 are carried out first. Thus, the user seizes the two portions 2, 3 of the girdle and draws them near his/her waist thereby partially encircling the latter, as shown in Figure 4. The user overlaps the free ends 2a, 3a of the girdle portions and actuates the removable fastening arrangement (of the Velcro® type, as described above). This will cause the girdle to encircle the waist of the user in a closed loop and to remain in position (see Figure 5). Then the user seizes the straps 14 and pulls them toward the overlapped ends of the girdle portions (see Figure 5). In this step, the straps act upon the pretensioning member/s 15, 16 (that were adjusted beforehand), and the latter will act upon the joining members 8, 9, that will stretch the pluralities of strips 4, 5. The pluralities of strips 4, 5 are stretched as controlled by the pretensioning member (i.e. to the same extent like in the configuration of Figure 1 or to different extents for the upper and lower portions of the girdle, like in the configuration of Figure 2). If the straps 14 are still pulled, the two girdle portions 2, 3 will be drawn further toward each other and thus allow the girdle to tightly fit on the user's body. Finally, the ends of the straps 14 are removably fastened to each other to maintain the tension so achieved (see Figure 6). Those skilled in the art will obviously appreciate that a number of changes and variants may be made to the above described configurations, to meet incidental and specific needs. For example, three or more groups of pluralities of strips may be provided for each portion of the girdle, with as many joining members corresponding thereto. In this case, the pretensioning members will include a corresponding number of straps, which are fastened together by a single joining element. Also, if four or more groups of strips are provided for each girdle portion, two pretensioning members may be provided for each girdle portion, which are acted upon by two corresponding straps. All of these variants and changes shall be contemplated in the scope of the invention, as defined in the following claims.

## Claims

1. Girdle comprising:
a first portion (2) and a second portion (3) each of which is in band form;
a first plurality of strips (4) that extend from the first portion (2) towards the second portion (3) and a second plurality of strips (5) that extend from the second portion (3) towards the first portion (2);
ends of the strips of each plurality of strips (4, 5 being collected into at least first (6) and second groups (7) each of which is joined to respective first (8) and second joining members (9);
tensioning members (12, 13) fixedly connected to the respective first (2) or second portion (3) in band form, able to be removably fastened to the second (3) or first portion (2) and active on said first (8) and second joining members (9) to allow a user to close said first (2) and second portion (3) in band form in a loop;
**characterised in that** it comprises pretensioning members (15, 16) each of which is designed to connect said first (8) or second joining members (9) to the respective tensioning members (12, 13), at least the pretensioning members (15, 16) that connect the first (8) or the second joining members (9) to the respective tensioning members (12, 13) being able to be removably fastened to the respective first (2) and second portion (3) in band form.

2. Girdle according to claim 1, wherein the pretensioning members (15, 16) that connect both the first (8) and the second joining members (9) to the respective tensioning members (12, 13) can be removably fastened to the respective first (2) and second portion (3) in band form.

3. Girdle according to claim 2, comprising indexing members (21) to fixedly connect said pretensioning members (15, 16) to the respective first (2) and second portion. (3) in band form in predefined positions.

4. Girdle according to claim 2 or 3, wherein each pretensioning member (15, 16) comprises a first portion (19) that slidably engages a respective tensioning member (12, 13), at least two intermediate portions that respectively slidably engage the first (8) and the second joining member (9) and at least two end portions (17a, 18a) able to be removably fastened to the first (2) or second portion (3) in band form.

5. Girdle according to claim 4, wherein each pretensioning member (15, 16) comprises a first (17) and a second strap (18), each of which is equipped with a free end (17a, 18a) defining one of said two end portions; said first (17) and second strap (18) being fixedly connected, at the opposite side with respect to the free ends (17a, 18a), to a respective joining element (19) defining said first portion of one of the pretensioning members (15, 16); a loop (20) being fixedly connected to said joining element (19) to slidably receive the tensioning member (12, 13).

6. Girdle according to claim 5, wherein said first (8) and second joining members (9) comprise respective loops (10, 11) engaged by a respective strap (17, 18) of said pretensioning members (15, 16); said loops (10, 11) engaging said straps (17, 18) in intermediate positions between the free ends (17a, 18a) and the joining element (19).

7. Girdle according to any one of the previous claims comprising a plurality of stiffening shafts (25) arranged between the first (2) or the second portion (3) in band form and said pretensioning members (15, 16) and/or said plurality of strips (4, 5).

8. Girdle according to any one of the previous claims, wherein each tensioning member (12, 13) comprises a strap (14) having a first end (14a). fixedly connected to the first (2) or second portion (3) in band form and a second end (14b) arranged to be actuated by a user; an intermediate portion of said strap (14) engaging a respective pretensioning member (15, 16).

9. Girdle according to any one of the previous claims, wherein said plurality of strips (4, 5), said pretensioning members (15, 16) and said tensioning members (12, 13) are made from flexible and non-elastic material.

10. Girdle according to any one of the previous claims, wherein said pretensioning members (15, 16) and said tensioning members (12, 13) are arranged, with respect to said portions (2, 3) in band form, on the opposite side with respect to the body of the user when the girdle is in use.

## Patentansprüche

1. Korsett umfassend:
einen ersten Abschnitt (2) und einen zweiten Abschnitt (3), die jeweils bandförmig sind;
eine erste Vielzahl von Bändern (4), die sich aus dem ersten Abschnitt (2) in Richtung des zweiten Abschnitts (3) erstrecken, und eine zweite Vielzahl von Bändern (5), die sich aus dem zweiten Abschnitt (3) in Richtung des ersten Abschnitts (2) erstrecken;
wobei die Enden einer jeden Vielzahl von Bändern (4, 5) in mindestens ersten (6) und zweiten Gruppen (7)gebündelt sind, die jeweils mit ersten (8) und zweiten Verbindungsgliedern (9) verbunden sind;
Spannglieder (12, 13), die fest mit dem jeweiligen ersten (2) oder zweiten bandförmigen Abschnitt (3) verbunden und in der Lage sind, abnehmbar mit dem zweiten (3) oder ersten Abschnitt (2) verbunden zu werden, und auf die ersten (8) und zweiten Verbindungsglieder (9) einwirken, um es einem Benutzer zu ermöglichen, den ersten (2) und den zweiten bandförmigen Abschnitt (3) in einer Schleife zu schließen;
**dadurch gekennzeichnet, dass** es Vorspannglieder (15, 16) umfasst, die jeweils dazu bestimmt sind, das erste (8) oder das zweite Verbindungsglied (9) mit den jeweiligen Spanngliedern (12, 13) zu verbinden, wobei mindestens die Vorspannglieder (15, 16), die das erste (8) oder das zweite Verbindungsglied (9) mit den jeweiligen Spanngliedern (12, 13) verbinden, in der Lage sind, abnehmbar mit den jeweiligen ersten (2) und zweiten bandförmigen Abschnitten (3) verbunden zu werden.

2. Korsett nach Anspruch 1, bei dem die Vorspannglieder (15, 16), die sowohl das erste (8) als auch das zweite Verbindungsglied (9) mit den jeweiligen Spanngliedern (12, 13) verbinden, abnehmbar an den jeweiligen ersten (2) und zweiten bandförmigen Abschnitten (3) befestigt werden können.

3. Korsett nach Anspruch 2, umfassend Indexierungsglieder (21), um die Vorspannglieder (15, 16) mit dem jeweiligen ersten (2) und zweiten bandförmigen Abschnitt (3) an zuvor festgelegten Positionen fest zu verbinden.

4. Korsett nach Anspruch 2 oder 3, bei dem jedes Vorspannglied (15, 16) einen ersten Abschnitt (19), der ein entsprechendes Spannglied (12, 13) verschiebbar in Eingriff nimmt, mindestens zwei Zwischenabschnitte, die jeweils das erste (8) und das zweite Verbindungsglied (9) verschiebbar in Eingriff nehmen, und mindestens zwei Endabschnitte (17a, 18a), die in der Lage sind, abnehmbar an dem ersten (2) oder zweiten bandförmigen Abschnitt (3) befestigt zu werden, umfasst.

5. Korsett nach Anspruch 4, bei dem jedes Vorspannglied (15, 16) ein erstes (17) und ein zweites Halteband (18) umfasst, die jeweils mit einem freien Ende (17a, 18a) versehen sind, die einen der beiden Endabschnitte definieren; wobei das erste (17) und zweite Halteband (18) an der den freien Enden (17a, 18a) gegenüberliegenden Seite fest mit einem jeweiligen Verbindungselement (19) verbunden sind, welches den ersten Abschnitt eines der Vorspannglieder (15, 16) definiert; wobei eine Schleife (20) fest mit dem Verbindungselement (19) verbunden ist, um das Spannglied (12, 13) verschiebbar aufzunehmen.

6. Korsett nach Anspruch 5, bei dem das erste (8) und das zweite Verbindungsglied (9) jeweilige Schleifen (10, 11) umfassen, die von einem jeweiligen Halteband (17, 18) der Vorspannglieder (15, 16) in Eingriff genommen werden; wobei die Schleifen (10, 11) die Haltebänder (17, 18) in Zwischenpositionen zwischen den freien Enden (17a, 18a) und dem Verbindungselement (19) in Eingriff nehmen.

7. Korsett nach einem der vorstehenden Ansprüche umfassend eine Vielzahl von Versteifungsstäben (25), die zwischen dem ersten (2) oder dem zweiten bandförmigen Abschnitt (3) und den Vorspanngliedern (15, 16) und/oder der Vielzahl von Bändern (4, 5) angeordnet sind.

8. Korsett nach einem der vorstehenden Ansprüche, bei dem jedes Spannglied (12, 13) ein Halteband (14) mit einem ersten, fest mit dem ersten (2) oder zweiten bandförmigen Abschnitt (3) verbundenes Ende (14a) und einem zweiten Ende (14b) umfasst, das angeordnet ist, um von einem Benutzer betätigt zu werden; wobei ein Zwischenabschnitt des Haltebands (14) ein entsprechendes Vorspannglied (15, 16) in Eingriff nimmt.

9. Korsett nach einem der vorstehenden Ansprüche, bei dem die Vielzahl von Bändern (4, 5), die Vorspannglieder (15, 16) und die Spannglieder (12, 13) aus flexiblem und unelastischem Material hergestellt sind.

10. Korsett nach einem der vorstehenden Ansprüche, bei dem die Vorspannglieder (15, 16) und die Spannglieder (12, 13) - bezogen auf die bandförmigen Abschnitte (2, 3) - auf der dem Körper des Benutzers gegenüberliegenden Seite angeordnet sind, wenn das Korsett getragen wird.

## Revendications

1. Ceinture comprenant :
une première portion (2) et une deuxième portion (3), chacune desquelles est sous forme de bande ;
une première pluralité de bandes (4) qui s'étendent de la première portion (2) vers la deuxième portion (3) et une deuxième pluralité de bandes (5) qui s'étendent de la deuxième portion (3) vers la première portion (2) ;
des extrémités des bandes de chaque pluralité de bandes (4, 5) étant rassemblées en au moins des premier (6) et deuxième groupes (7), chacun desquels est uni à des premier (8) et deuxième éléments d'assemblage (9) respectifs ;
des éléments de tension (12, 13) connectés de manière fixe à la première (2) ou deuxième portion (3) respective sous forme de bande, adaptés pour être attachés de manière amovible à la deuxième (3) ou première portion (2) et actifs sur lesdits premier (8) et deuxième éléments d'assemblage (9) pour permettre à un utilisateur de fermer ladite première (2) et deuxième portion (3) sous forme de bande en une boucle ;
**caractérisée en ce qu'**elle comprend des éléments de précontrainte (15, 16), chacun desquels est conçu pour connecter lesdits premier (8) ou deuxième éléments d'assemblage (9) aux éléments de tension (12, 13) respectifs, au moins les éléments de précontrainte (15, 16) qui connectent le premier (8) ou le deuxième éléments d'assemblage (9) aux éléments de tension (12, 13) respectifs étant adaptés pour être attachés de manière amovible à la première (2) et la deuxième portion (3) sous forme de bande respective.

2. Ceinture selon la revendication 1, dans laquelle les éléments de précontrainte (15, 16) qui connectent à la fois le premier (8) et le deuxième élément d'assemblage (9) aux éléments de tension (12, 13) respectifs peuvent être attachés de manière amovible à la première (2) et la deuxième portion (3) sous forme de bande respective.

3. Ceinture selon la revendication 2, comprenant des éléments d'indexage (21) pour connecter de manière fixe lesdits éléments de précontrainte (15, 16) à la première (2) et la deuxième portion (3) sous forme de bande respective dans des positions prédéfinies.

4. Ceinture selon la revendication 2 ou 3, dans laquelle chaque élément de précontrainte (15, 16) comprend une première portion (19) qui engage de manière coulissante un élément de tension (12, 13) respectif, au moins deux portions intermédiaires qui engagent de manière coulissante respectivement le premier (8) et le deuxième élément d'assemblage (9) et au moins deux portions d'extrémité (17a, 18a) adaptées pour être attachées de manière amovible à la première (2) ou deuxième portion (3) sous forme de bande.

5. Ceinture selon la revendication 4, dans laquelle chaque élément de précontrainte (15, 16) comprend une première (17) et une deuxième sangle (18), chacune desquelles est équipée d'une extrémité libre (17a, 18a) définissant une desdites deux portions d'extrémité ; ladite première (17) et ladite deuxième sangle (18) étant connectée de manière fixe, au niveau d'un côté opposé par rapport aux extrémités libres (17a, 18a), à un élément d'assemblage (19) respectif définissant ladite première portion d'un des éléments de précontrainte (15, 16) ; une boucle (20) étant connectée de manière fixe audit élément d'assemblage (19) pour recevoir de manière coulissante l'élément de tension (12, 13).

6. Ceinture selon la revendication 5, dans laquelle lesdits premier (8) et deuxième éléments d'assemblage (9) comprennent des boucles (10, 11) respectives engagées par une sangle (17, 18) respective desdits éléments de précontrainte (15, 16) ; lesdites boucles (10, 11) engageant lesdites sangles (17, 18) dans des positions intermédiaires entre les extrémités libres (17a, 18a) et l'élément d'assemblage (19).

7. Ceinture selon l'une quelconque des revendications précédentes, comprenant une pluralité de tiges de raidissement (25) agencées entre la première (2) ou la deuxième portion (3) sous forme de bande et lesdits éléments de précontrainte (15, 16) et/ou ladite pluralité de bandes (4, 5).

8. Ceinture selon l'une quelconque des revendications précédentes, dans laquelle chaque élément de tension (12, 13) comprend une sangle (14) ayant une première extrémité (14a) connectée de manière fixe à la première (2) ou deuxième portion (3) sous forme de bande et une deuxième extrémité (14b) agencée pour être actionnée par un utilisateur ; une portion intermédiaire de ladite sangle (14) engageant un élément de précontrainte (15, 16) respectif.

9. Ceinture selon l'une quelconque des revendications précédentes, dans laquelle ladite pluralité de bandes (4, 5), lesdits éléments de précontrainte (15, 16) et lesdits éléments de tension (12, 13) sont réalisés à partir d'un matériau flexible et non élastique.

10. Ceinture selon l'une quelconque des revendications précédentes, dans laquelle lesdits éléments de précontrainte (15, 16) et lesdits éléments de tension (12, 13) sont agencés, par rapport auxdites portions (2, 3) sous forme de bande, sur le côté opposé par rapport au corps de l'utilisateur quand la ceinture est utilisée.
